# EUROPEAN PATENT APPLICATION

(11) **EP 4 349 283 A1**
(43) Date of publication of application: **10.04.2024**
(21) Application number: 22306470.0
(22) Date of filing: 04.10.2022
(51) Int. Cl.: A61B 17/22, A61B 8/00, A61N 7/00, A61B 17/00

(54) **COMPUTER-IMPLEMENTED METHOD FOR CONTROLLING THE OPERATION OF AN ULTRASOUND APPARATUS**

(71) Applicant: Cardiawave, 75017 Paris (FR)
(72) Inventor: GRACIANO FOUQUIER, Ana Beatriz, 75013 PARIS (FR); BRAND, Alexandre, 91300 MASSY (FR)
(74) Representative: Atout PI Laplace

(57) **Abstract**

The invention relates to a computer-implemented method for controlling the operation of an apparatus able to produce ultrasound waves focused inside a target and to obtain at least one image of the target, the method comprising determining or receiving from a user and/or a memory a range of allowed acoustic power levels for the apparatus as a function of the target and at least one configuration parameter of the apparatus; displaying on a display a user interface, detecting at least one activation of any control element of the user interface by a user, and transmitting at least one instruction to the apparatus based on the activation. The invention also relates to such a computer system, a computer program product for carrying out the method and a non-transitory computer-readable data-storage medium containing computer-executable instructions to cause a computer system to carry out the method.

## Description

### FIELD OF THE INVENTION

The invention relates to a computer-implemented method for controlling the operation of an apparatus able to produce ultrasound waves focused inside a target and to obtain at least one image of the target.

The invention also relates to a computer system in communication with such apparatus comprising one or more programs including instructions for performing the said method. Further, the invention relates to a computer program product, stored on a non-transitory computer-readable data-storage medium comprising computer-executable instructions to cause such computer system to carry out said method and to such non-transitory computer-readable data-storage medium.

The invention applies to both industrial and medical applications of ultrasound. In the following, the focus will be on medical - and more specifically, therapeutic application - applications, but this shall not be considered limitative.

### BACKGROUND PRIOR ART

Interest in technologies using ultrasonic waves has grown steadily as numerous potential applications have been discovered. Ultrasonic waves may be used to induce cavitation bubbles by focusing ultrasound pulses at a focal spot, allowing the release of mechanical energy towards a specific target in a region included, partially included or close to the focal spot. Multiple cavitation bubbles may be generated in such region close to or at the focal spot, which may therefore be identified as a cavitation region. The cavitation bubbles induced may be referred to as a cavitation cloud.

Cavitation bubbles or cloud generated by ultrasonic waves may be used for many different applications. Regarding medical applications, cavitation bubbles may be used for instance for histotripsy (the mechanical destruction of tissues), thrombotripsy (mechanical destruction of thrombi) or lithotripsy (fragmentation of calculi). Such applications have the particular advantage of being non- or minimally-invasive. There are many other uses for ultrasound waves, such as in sonochemistry which may result in the initiation or enhancement of the chemical activity in a solution, in ultrasonic cleaning, ultrasonic disintegration and many others.

For instance, during histotripsy, an acoustic transducer generates short bursts of sinusoidal ultrasound waves that will focus in the target zone, usually working at a central emission frequency between 700 kHz and 4 MHz, with 1 to 20 repeated cycles per burst at a pulse-repetition frequency between 1 and 1000 Hz. Histotripsy locally produces pressures of up to 100 MPa and down to -25 MPa.

Some applications require focused ultrasound pulses with a high level of precision regarding the focal spot and the cavitation region where the cavitation bubbles or cloud will be generated, especially with medical applications. Controlling the position and/or the dimensions of the cavitation region remains challenging as the expected cavitation region is relatively uncertain according to the target and to the apparatus. It is particularly important to ensure that the cavitation region is the closest to the target. Typically, the ultrasound wave apparatus also comprises means for obtaining an image of the target, which allow identifying the cavitation and where cavitation bubbles are generated.

The acoustic power level of the apparatus allows the generation of cavitation bubbles above a particular threshold, which is dependent on many parameters such as the apparatus, the distance to the target, as well as on environmental parameters. On the contrary, too high an acoustic power level can lead to cavitation bubbles being generated in undesired locations, or to too many cavitation bubbles. In order to control an ultrasound wave apparatus as accurately as possible the acoustic power level should exceed the cavitation threshold while not exceeding a safety limit at the same time.

These thresholds and limits depend on many parameters, such as the target composition or size, the hardware elements of the apparatus which allows modulating the acoustic power level, the shape of the transducers for emitting ultrasound waves, the environmental parameters, the acoustic coupling between system and the object to be insonicated, etc. These parameters may be unstable and vary in time. In order to ensure the finest possible control, a user may try to increase or decrease an acoustic power level while monitoring the generation of cavitation bubbles. For medical applications, the acoustic output power level, or acoustic power level, may correspond to a "therapy gain value".

However using such an apparatus with the finest possible control requires extensive preparations and a large number of simultaneous actions. Indeed, the user must be able to prepare the ultrasound apparatus and position it regarding the target, predetermine a specific acoustic power level for the ultrasound apparatus so as to generate cavitation, activate the apparatus at the right time, verify the normal operation of the apparatus following such activation, increase the acoustic power level until cavitation bubbles are generated and seen at the same time, maintain an effective acoustic power level while verifying that the cavitation bubbles are still generated at the target and without an acoustic power level exceeding the safety limits. Indeed, the objective for the user is to determine the lowest therapy gain (as low as reasonably achievable) that allows triggering the cavitation phenomenon, a.k.a. "cavitation threshold". Determining such lowest therapy gain is a difficult task as it depends on multiple factors. Notably, there is no standard value that fits for all subjects and, for a given subject there is no standard value that fits for all target zones and for the duration of the entire insonification session. Determining such lowest therapy gain is a difficult task as it depends on multiple factors.

All these steps require for the user an intense concentration, a short reaction time and an important mental workload, which the user may try to manage by taking their time during preparation of any action, while it is a procedure that one wishes to keep as short as possible, for efficiency reasons, energy saving, safety reasons (for medical applications) and/or for preserving the insonified materials (in industrial applications).

Also, the user needs to rely on the real time images to monitor the cavitation effect of focused ultrasound waves generated by the ultrasound apparatus. The user may also identify audible noise to adjust the appropriate acoustic power level. Simultaneously, in medical applications, the user may also need to monitor at least one subject vital sign simultaneously, which also requires additional attention.

The known hardware solutions (footswitches, knobs, or joysticks) present several drawbacks in this context. They require the user to be actively controlling the increment/decrement of acoustic power level of the apparatus through repetitive body movements, which can provoke injuries (repetitive stress injuries) in the long term, especially when an ultrasound session may last for several minutes and a complete insonification session may last for an hour.

They are inherently limited in their responsiveness to the user action, due to the way they are built. Unlike their use in High Intensity Focused Ultrasound (HIFU), which induces thermal effects on a long temporal scale and whose heat map progresses slowly, they may be too difficult to control for cavitation treatment by ultrasound, such as histotripsy or image-guided Non-Invasive Ultrasound Therapy (NIUT) which has a fast mechanism of action with very short reaction time of the human anatomy (e.g., heart arrythmias) and requires surveillance and precautions from users.

Users may also suffer from physical fatigue, which could impair the effectiveness/efficacy of the sessions and the choice of an adapted acoustic power level of the apparatus. Furthermore, the user cannot convey all the visual feedback about the current acoustic power level and require another user interface support to convey the remaining required information.

Known Graphical User Interfaces (GUI) only have a limited behavior scope (simple click, toggle mode, long press), and cannot convey the full information about the Intensity Spatial Peak Pulse Average (I_{SPPA}) and the Therapy Gain value ranges.

There is therefore the need for means or a method which help the user to ensure the finest possible control of the ultrasound wave apparatus while lightening the mental workload of the user when using the ultrasound waves apparatus.

### SUMMARY OF THE INVENTION

The invention aims at overcoming in full or in part the aforementioned drawbacks of the prior art. More particularly, it aims to help the user to ensure fine control of a ultrasound wave apparatus while reducing the number of interactions between the user and the apparatus.

The invention relates to a computer-implemented method for controlling the operation of an apparatus able to produce ultrasound waves focused inside a target and to obtain at least one image of the target, the method comprising:
- determining or receiving from a user and/or a memory a range of allowed acoustic power levels for the apparatus as a function of the target and at least one configuration parameter of the apparatus;
- displaying, on the display a user interface including at least:
   ∘ a first area displaying at least one image of the target,
   ∘ a second area displaying a control element for activating or stopping
   the production of ultrasound waves by the apparatus,
   ∘ a third area displaying a current acoustic power level emitted by the apparatus, the determined range of allowed acoustic power levels and a control element for selecting any acoustic power level within the range of allowed acoustic power levels,
   wherein the third area includes at least one control element for increasing progressively and automatically the acoustic power level of the apparatus by selection of a higher value, the progressive and automatic evolution following a predetermined speed law;

while displaying the user interface, detecting at least one activation of any control element of the user interface by a user, and
transmitting at least one instruction to the apparatus, wherein the instruction corresponds to the at least one activation of any control element of the user interface.

By "detecting at least one activation of any control element of the user interface by a user", it is meant that the activation may be performed by an interface device, such interface device being for example a computer mouse, trackball, joystick, a physical control element or button or the screen itself which is then a tactile screen or any other physical object interfacing an action of the user with the activation of a control element. If the interface device is not the screen itself, it is preferably arranged next to the screen.

Advantageously, the third area may include at least one control element for decreasing progressively and automatically the acoustic power level of the apparatus by selection of a lower value, the progressive and automatic evolution following a predetermined speed law, such control element being the same or a different element from the at least one element for increasing progressively and automatically the acoustic power level of the apparatus.

Advantageously, the progressive and automatic evolution of the acoustic power level of the apparatus may follow a first speed law when increasing and a second speed law when decreasing, the first and the second speed laws being different. Alternatively or complementarily, the decrease to a specific lower value may be instantaneous.

Advantageously, the apparatus may comprise a power amplifier and the range of allowed acoustic power levels is further adapted according to the temperature of the power amplifier or other hardware components. Further, the range of acoustic power levels may be adapted to any factor which would limit the maximal acoustic power level. For instance, especially in medical applications, a limitation of the cumulated dose may intervene in the adaptation of the range of acoustic power level. Furthermore, in medical application, the range of acoustic power levels may be restricted to acceptable values according to patient BMI (Body Mass Index), allowing to adapt to different acoustic attenuation factors due to different patient thicknesses. Moreover, the range of acoustic power levels may depend on the physical phenomena (e.g.: mechanical, thermal) to be induced by the ultrasounds. For example, in clinical applications, the treatment of a Calcified Aortic Valve requires a lower level of acoustic power (objective is to soften the tissues) compared to an Histotripsy Ablation application (objective is to destroy cancerous cells).

Advantageously, a threshold of temperature for the power amplifier may be predetermined and the user interface may then display a first alert when the threshold of temperature is close to the temperature of the power amplifier and/or a second alert when the threshold of temperature is exceeded. By "close", it is meant that the temperature of the power amplifier reaches a value which corresponds for instance to 90 %, 95 % or 98 % of the threshold of temperature. The user may choose what percentage is the most appropriate based on at least one of the configuration parameters and/or the target.

Advantageously, the user interface displays a cumulated dose corresponding to the cumulated focused waves produced by the apparatus at the target site and wherein the range of allowed acoustic power levels is further adapted according to a predetermined maximum cumulated dose.

The invention also relates to a computer system in communication with an apparatus, the apparatus being able to produce ultrasound waves focused inside a target and to obtain at least one image of the target, wherein the computer system comprises: a display, one or more processors, a memory, and one or more programs, wherein the one or more programs are stored in the memory and configured to be executed by the one or more processors, the one or more programs including instructions for performing a method for controlling the operation of the apparatus of the invention.

Advantageously, the display of the computer system may comprise a touch-sensitive surface and the user interface is displayed on the touch-sensitive surface, the touch-sensitive surface being able to detect intensities of contacts with the touch-sensitive surface. Alternatively, it may comprise a non touch-sensitive ("regular") screen, in which case user actions are performed through a device such as a mouse or joystick.

The invention also relates to a computer program product, stored on a non-transitory computer-readable data-storage medium comprising computer-executable instructions to cause a computer system to carry out a method for controlling the operation of the apparatus of the invention.

The invention also relates to a non-transitory computer-readable data-storage medium containing computer-executable instructions to cause a computer system to carry out a method for controlling the operation of the apparatus of the invention.

### BRIEF DESCRIPTION OF THE FIGURES

The invention will be better understood and its various characteristics and advantages will emerge from the following description of a number of exemplary embodiments and its appended figures in which:

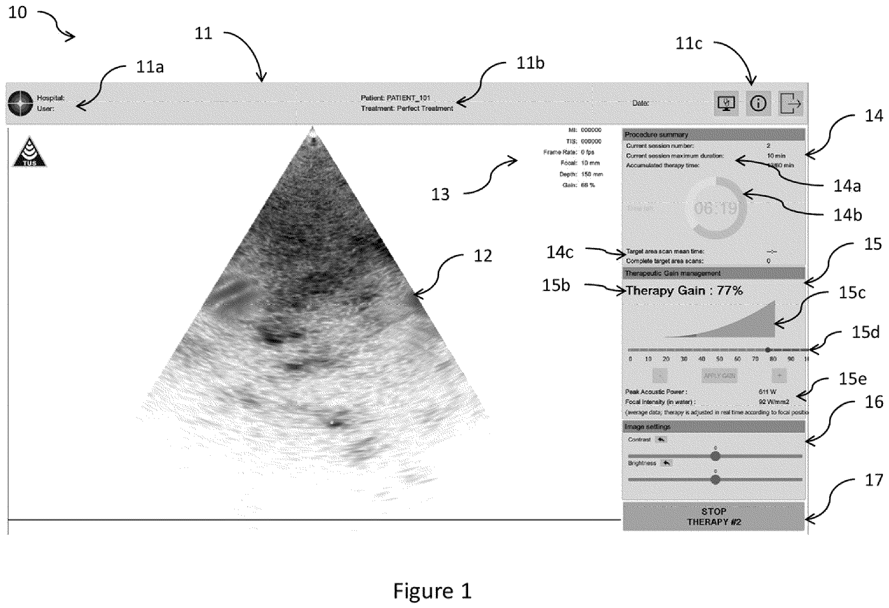

Figure 1 displays a first user interface which may be displayed on the display of a computer system in communication with an apparatus able to produce ultrasound waves focused inside a target and to obtain at least one image of the target, and which is used to perform the computer-implemented method of the invention.

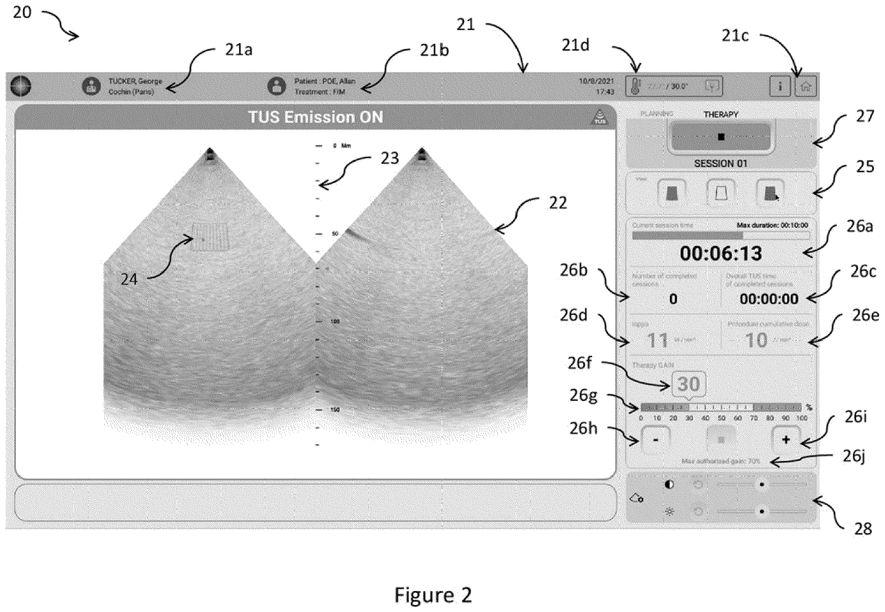

Figure 2 displays a second user interface which may be displayed on the display of a computer system in communication with an apparatus able to produce ultrasound waves focused inside a target and to obtain at least one image of the target, and which is used to perform the computer-implemented method of the invention.

### DETAILED DESCRIPTION OF THE INVENTION

In this specification, the invention will be further described, notably by way of examples demonstrating reduction to practice of the computer-implemented method of the invention. However, the invention is not restricted to these examples.

The computer-implemented method of the present invention allows the automation of ultrasound wave sessions. Notably, thanks to the progressive and automatic increase and potentially the progressive and automatic decrease, the user interface can transmit information for adapting the acoustic power level to a targeted value in one activation of a control element, allowing the user to monitor in real time the ultrasound images during the evolution of the acoustic power level, without having to focus on the selection of the acoustic power level. This also contributes to the user interface usability and improvement of the user comfort.

The real time control of the acoustic power level ensures that the Intensity Spatial Peak Pulse Average (I_{SPPA}) increases or decreases in an optimal lapse of time as it is controlled.

The computer implemented method of the present invention is safer than the method of the prior art as the user is able not only to stop the progression while monitoring in real time the images without having to manipulate other elements at the same time but also to set immediately a lower/safer acoustic power level if needed at the same time.

Also, thanks to the speed law in the progressive and automatic increase, the acoustic power level does not reach higher I_{SPPA} values immediately, allowing the user to monitor the subject responses to the acoustic power level increase, and to stop its progression if needed to stop completely the apparatus or to reduce the acoustic power level to a reduced value.

The present invention further allows for adjusting the I_{SPPA} values to account for any technical constraints (hardware, I_{SPPA} dose). For instance, if the temperature in the power amplifier linked to the ultrasound transducer is reaching a threshold, the determined range of acoustic power levels is adapted and this is visible immediately by the user in the user interface.

If after a given session duration the cumulated dose has not reached a given threshold, the determined range of allowed acoustic power levels may be adapted by limiting its minimum acoustic power level to optimize the efficacy of the procedure. Also the present invention allows the user to perform a specific session of ultrasound waves in a shorten time.

By "cumulated dose", it is meant the sum of the intensities that the apparatus delivered at the target sites throughout the time (while producing ultrasound waves).

Figure 1 displays a first user interface which may be displayed on the display of a computer system in communication with an apparatus able to produce ultrasound waves focused inside a target and to obtain at least one image of the target, and which is used to perform the computer-implemented method of the invention.

The first user interface of figure 1 comprises a first area 12 displaying an image of the target. The image of the target displayed is obtained by the apparatus able to produce ultrasound waves focused inside the target. The image may be obtained via any imaging technology, although an ultrasound imaging transducer is preferred for the present invention. Although the image displayed has a specific shape on figure 1, the image displayed may have any shape. Although the image displayed is in black and white, the image may also be displayed in color, or only partially in color. In the case of the figure, the target to be insonicated corresponds to the whole image.

In particular embodiments which may be combined to any other embodiment, not requiring any particular adaptation of the other embodiments, the first area may display more than one image of the target.

The user interface of figure 1 also displays a second area displaying a control element 17 for activating or stopping the production or emission of focused ultrasound waves by the apparatus. According to figure 1, the control element 17 allows stopping the apparatus wave emission.

Although not shown, control element 17 may be used to stop the apparatus when the apparatus is emitting ultrasound waves and the same control element 17 may be used to activate the apparatus when it is not emitting ultrasound waves.

Alternatively, control element 17 of the second area comprises two parts, one for activating the apparatus and one for stopping the apparatus. Control element 17 may have any shape or color, and may shift color depending on the status of the apparatus (activated or stopped).

The user interface of figure 1 also includes a third area 15 displaying a current acoustic power level emitted by the apparatus. The current acoustic power level may be displayed by a value 15b and/or by a gauge 15c and/or by a scale 15d. Other symbols or ways of displaying the current acoustic power level may be used. The third area 15 also displays the determined range of the acoustic power level, such as with the gauge 15c or with a scale 15d. The gauge 15c or the scale 15d may have particular combinations of colors to indicate the current acoustic power level and/or the determined range of allowed acoustic power level.

The third area also displays a control element for selecting any acoustic power level within the range of allowed acoustic power levels. Such control element may correspond to the gauge 15c where the user may click directly on the gauge 15c to choose the acoustic power level of the apparatus, or it may correspond to the scale 15d, where the user may click directly on any part of the scale to choose the acoustic power level of the apparatus. Other designs of this control element may be used with the present invention.

The third area may also comprise control elements for increasing and optionally decreasing the acoustic power level by unitary steps, such as simple square buttons as shown in figure 1 under the scale 15d and above the data displayed 15e, which are data related to the parameters of the ultrasound apparatus.

By "unitary steps" it is meant any fixed value or any percentage of a value, such as for instance unitary steps of watts, or a percentage of the determined range of acoustic power level, or a percentage of the maximum acoustic power level of the apparatus. For instance, such fixed value may correspond to a percentage of the maximal acoustic power level, for example 1 %, 2 %, 3 %, 5 %, 10 %, 20 % or any other value. The user may define what the most appropriate percentage is based on the application. Also, a minimal value of percentage of the maximal acoustic power level may be set, such as for instance 1 %, 2 %, 5 %, 10 %, 20 % or any other value. Preferably the minimal acoustic power level percentage is set at a percentage value which is 20 % or more to 30 % included. The unitary step may differ when increasing or when decreasing.

The control element for selecting the acoustic power level of the apparatus, such as gauge 15c or scale 15d may correspond to a control for increasing and optionally decreasing progressively and automatically the acoustic power level of the apparatus by selection of a higher or a lower value than the current acoustic power level of the apparatus, where the progressive and automatic evolution of the acoustic power level follows a predetermined speed law. Such predetermined speed law may be chosen by the user before or during the use of the method of the invention. Information regarding the peak acoustic power and the focal intensity 15e is also presented.

For instance, the progressive and automatic evolution of the acoustic power level of the apparatus, would it be an increase or a decrease, may follow a predetermined speed law among at least a linear, exponential, logarithmic, quadratic, or a cubic law. In fact, the progressive and automatic evolution may follow any function which may be deployed as a law.

The speed law for increasing the acoustic power level of the apparatus may follow a different speed law than the speed law for decreasing the acoustic power level of the apparatus. The user is able to define which speed law suits better the increase and/or the decrease of the acoustic power level and to select which speed law should be applied when the control element for selecting an acoustic power level is activated.

The user interface of figure 1 displays a general information area 11 which indicates multiple data such as, for instance, the hospital and user name number 11a, the patient name or number and the treatment planned 11b, and control elements 11c for setting the user interface or closing the user interface. An image setting area 16 also allows modifying the brightness and contrast of the image. This general information area 11 and image setting area may comprise any other information and is not essential for performing the computer implemented method of the invention.

The user interface of figure 1 also comprises imaging information 13 regarding the image displayed, such as the frame rate, the depth, the focal, or any other parameter. The imaging information 13 is not essential for performing the computer implemented method of the invention.

The user interface of figure 1 displays a procedure summary area 14 which displays session information 14a comprising, for instance, the current session number, the current session maximum duration and the accumulated therapy time. Further, the procedure summary area 14 displays the time left 14b for using the ultrasound transducer apparatus. Also, the procedure summary area 14 displays the target area scan mean time and the number of complete target area scans 14c. This information is not essential for performing the computer implemented method of the invention.

Figure 2 displays a second user interface which may be displayed on the display of a computer system in communication with an apparatus able to produce ultrasound waves focused inside a target and to obtain at least one image of the target, and which is used to perform the computer-implemented method of the invention.

The user interface of figure 2 comprises a first area 22 displaying an image of the target. In figure 2, the image of the target comprises two parts. The two parts may be obtained from a different angle of the imaging transducer. Although the image displayed has a specific shape on figure 2, the image displayed may have any shape. Although the image displayed is in black and white, the image may also be displayed in color, or only partially in color.

In the user interface of figure 2, the image is covered by a layer comprising a scale 23 indicating the depth and a pattern 24 in the shape of a grid adapted to the target as the target is represented by a dot inside the perimeter of the grid. The pattern allows the user to faster identify where the target is, or where the expected focus of the waves emitted by the ultrasound transducer. Furthermore, the color or the shape of the pattern may change according to the acoustic power level of the ultrasound transducer or according to any other parameter. Alternatively, the pattern may take any shape or color, depending on the user choice and/or the target and/or the image obtained. Preferably, the shape of the pattern is adapted to be around or over an expected focus spot of the ultrasound waves produced by the apparatus. For instance, the pattern may correspond to at least one circle, square, or any other shape which is adapted to the target, optionally depending on the acoustic power level of the ultrasound transducer. The use of such a pattern - either in the shape of a grid or not - may be combined to any other embodiment, not requiring any particular adaptation of the other embodiments.

The user interface of figure 2 also displays a view area 25 that allows switching between different views for image 22 and to add or remove at least one pattern, such as pattern 24.

The user interface of figure 2 displays a general information area 21 which indicates multiple data such as the hospital and user name 21a, the patient name or number and the treatment planned 21b, and control elements 21c for setting the user interface or closing the user interface. The general information area also comprises an indicator 21d of the temperature of the amplifier. An image setting area 28 allows modifying the brightness and contrast of the image. This general information area 21 and image setting area may comprise any other information and is not essential for performing the computer implemented method of the invention.

The user interface of figure 2 displays a second area displaying a control element 27 for activating or stopping the production or emission of focused ultrasound waves by the apparatus. According to figure 2, the control element 27 allows stopping the apparatus wave emission. Although not shown, control element 27 may be used to stop the apparatus when the apparatus is emitting ultrasound waves and the same control element 27 may be used to activate the apparatus when it is not emitting ultrasound waves.

The user interface of figure 2 also includes a third area 26 displaying a current acoustic power level emitted by the apparatus. The current acoustic power level may be displayed by a value 26f and/or by a gauge or scale 26g. Other symbols or ways of displaying the current acoustic power level may be used. The third area 26 also displays the current session time 26a, the number of completed sessions 26b, the overall time of completed sessions 26c, the Intensity Spatial Peak Pulse Average (I_{SPPA}) (at focal spot, an extrapolated value either in water or in a human subject), the procedure cumulative dose 26e, and the maximum authorized acoustic power level 26j.

The third area displays control elements for selecting any acoustic power level within the range of allowed acoustic power levels. These control elements may correspond to the gauge 26g where the user may click directly on the gauge 26g to choose the acoustic power level of the apparatus. Other designs of the control elements may be used with the present invention.

The third area of figure 2 also comprises control elements 26h, 26i for increasing or decreasing the acoustic power level by unitary steps, such as simple square buttons 26h and 26i.

The control element for selecting the acoustic power level of the apparatus, such as gauge 26g may correspond to a control element for increasing or decreasing progressively and automatically the acoustic power level of the apparatus by selection of a higher or a lower value than the current acoustic power level of the apparatus, where the progressive and automatic evolution of the acoustic power level follows a predetermined speed law. Such predetermined speed law may be chosen by the user before or during the use of the method of the invention.

The various embodiments presented in this description are not limiting and may be combined with each other. Furthermore, the present invention is not limited to the previously described embodiments but extends to any embodiment within the scope of the claims.

## Claims

1. A computer-implemented method for controlling the operation of an apparatus able to produce ultrasound waves focused inside a target and to obtain at least one image of the target, the method:
- determining or receiving from a user and/or a memory a range of allowed acoustic power levels for the apparatus as a function of the target and at least one configuration parameter of the apparatus;
- displaying, on the display a user interface including at least:
∘ a first area (12, 22) displaying at least one image of the target,
∘ a second area displaying a control element (17, 27) for activating or stopping the production of ultrasound waves by the apparatus,
∘ a third area (15, 26d, 26e, 26f) displaying a current acoustic power level emitted by the apparatus, the determined range of allowed acoustic power levels and at least one control element (15c, 15d, 26g) for selecting any acoustic power level within the range of allowed acoustic power levels,
wherein at least one said control element (15c, 15d, 26g) displayed in the third area is configured for increasing progressively and automatically the acoustic power level of the apparatus by selection of a higher value, the progressive and automatic evolution following a predetermined speed law;
- while displaying the user interface, detecting at least one activation of any control element of the user interface by a user, and
- transmitting at least one instruction to the apparatus, wherein the instruction corresponds to the at least one activation of any control element of the user interface.

2. The method of claim 1, wherein at least one said control element displayed in the third area is configured for decreasing progressively and automatically the acoustic power level of the apparatus by selection of a lower value, the progressive and automatic evolution following a predetermined speed law, such control element being the same or a different element from the at least one element for increasing progressively and automatically the acoustic power level of the apparatus.

3. The method of claim 2, wherein the progressive and automatic evolution of the acoustic power level of the apparatus follows a first speed law when increasing and a second speed law when decreasing, the first and the second speed laws being different.

4. The method of any of the preceding claims, wherein the apparatus comprises a power amplifier and wherein the range of allowed acoustic power levels is further adapted according to the temperature of the power amplifier.

5. The method of claim 4, wherein a threshold of temperature for the power amplifier is predetermined and wherein the user interface displays a first alert when the threshold of temperature is close and/or a second alert when the threshold of temperature is exceeded.

6. The method of any of the preceding claims, wherein the user interface displays a cumulated dose corresponding to the cumulated focused waves produced by the apparatus at the target site and wherein the range of allowed acoustic power levels is further adapted according to a predetermined maximum cumulated dose.

7. A computer system in communication with an apparatus, the apparatus being able to produce ultrasound waves focused inside a target and to obtain at least one image of the target, the computer system comprising: a display, one or more processors, a memory, and one or more programs, wherein the one or more programs are stored in the memory and configured to be executed by the one or more processors, the one or more programs including instructions for performing, when executed by the computer system, a method for controlling the operation of the apparatus according to any of claims 1-6.

8. The computer system of claim 7, wherein the display comprises a touch-sensitive surface and the user interface is displayed on the touch-sensitive surface, the touch-sensitive surface being able to detect intensities of contacts with the touch-sensitive surface.

9. A computer program product, stored on a non-transitory computer-readable data-storage medium comprising computer-executable instructions to cause a computer system to carry out a method for controlling the operation of the apparatus according to any of claims 1-6.

10. A non-transitory computer-readable data-storage medium containing computer-executable instructions to cause a computer system to carry out a method for controlling the operation of the apparatus according to any of claims 1-6.
